# EUROPEAN PATENT APPLICATION

(11) **EP 4 364 905 A1**
(43) Date of publication of application: **08.05.2024**
(21) Application number: 21948298.1
(22) Date of filing: 29.06.2021
(51) Int. Cl.: B25J 13/00

(54) **ROBOT DEVICE AND METHOD FOR OPERATING ARM**

(71) Applicant: Fuji Corporation, Chiryu-shi, Aichi 472-8686 (JP)
(72) Inventor: YAMASHITA, Yasuhiro, Chiryu-shi, Aichi 472-8686 (JP); HIGASHI, Shogo, Chiryu-shi, Aichi 472-8686 (JP); ISHIKAWA, So, Chiryu-shi, Aichi 472-8686 (JP)
(74) Representative: Grünecker Patent- und Rechtsanwälte PartG mbB
(86) International application number: PCT/JP2021/024542
(87) International publication number: WO 2023/275989

(57) **Abstract**

A robot device includes an arm holding a probe of an ultrasonic diagnostic device and a force sensor provided on the arm, supports a manual operation of the probe by an operator based on a signal from the force sensor, operates the arm to automatically operate the probe based on an instruction of the operator, and includes a storage device and a control device. The storage device stores a position and a posture of the probe for each point among multiple random points by the manual operation. The control device interpolates a trajectory between the multiple points stored in the storage device, and operates the arm such that the probe moves along the trajectory in the automatic operation and can stop at a desired position between the multiple points based on the instruction of the operator.

## Description

### Technical Field

The present description discloses a robot device and an arm operating method.

### Background Art

Conventionally, a robot device is known in which an ultrasonic probe is held by a robot arm and the robot arm is operated to scan a body surface of a subject. For example, Patent Literature 1 discloses an ultrasonic diagnostic device including a robot arm that holds an ultrasonic probe, a storage section that stores instruction trajectory information for moving the ultrasonic probe by the robot arm, and a control section that controls drive of the robot arm so as to move the ultrasonic probe according to the stored instruction trajectory information. The instruction trajectory information is generated based on reference trajectory information obtained by an operator that manually moves the ultrasonic probe held by the robot arm, and the reference trajectory information includes information, such as a position, an inclination, a movement path, a movement speed, and a living body contact pressure of the ultrasonic probe. Furthermore, the instruction trajectory information is generated as a smoother line by linearly approximating time-series data of a movement speed and time-series data of an inclination included in the reference trajectory information by the least squares method or approximating the time-series data by a curve of a predetermined order. The control section performs a feedback control of the robot arm such that the ultrasonic probe moves according to the instruction trajectory information by using the instruction trajectory information and a detection signal from each arm sensor. Further, the ultrasonic diagnostic device also provides a manual movement mode in which an operator can manually move the ultrasonic probe, and a manual support mode for providing supports, such as supporting a weight of a probe, keeping a probe movement speed constant, suppressing probe shake, and keeping a living body contact pressure constant when an operator moves the ultrasonic probe.

### Patent Literature

Patent Literature 1: JP-A-2017-159027

### Summary of the Invention

### Technical Problem

In the above-described robot device, a robot arm is operated such that an ultrasonic probe is moved by interpolating between respective points based on instruction movement information obtained for each point by manually moving the ultrasonic probe held by the robot arm by an operator. However, when diagnosis is performed, in some cases, it may be necessary to stop the ultrasonic probe at an essential place or move the ultrasonic probe slowly, and simply automatically moving the ultrasonic probe as an operator manually moves the ultrasonic probe in advance may lack convenience.

A main object of the present disclosure is to improve convenience of probe diagnosis using a robot device.

### Solution to Problem

The present disclosure employs the following means to achieve the main object described above.

A robot device of the present disclosure includes an arm holding a probe of an ultrasonic diagnostic device and a force sensor provided on the arm, the robot device being for supporting a manual operation of the probe by an operator based on a signal from the force sensor and operating the arm to automatically operate the probe based on an instruction of the operator, a storage device configured to store a position and a posture of the probe for each point among multiple random points by the manual operation, and a control device configured to interpolate a trajectory between the multiple points stored in the storage device, and operate the arm such that the probe moves along the trajectory in the automatic operation and can stop at a desired position between the multiple points based on the instruction of the operator.

A robot device of the present disclosure stores a position and posture of a probe for each point at multiple random points by manual operation. Then, the robot device interpolates a trajectory between the multiple points, and the probe moves along the trajectory in an automatic operation. Accordingly, the probe can be automatically operated to acquire an ultrasonic image of the inside of the body of a subject, and an operator can treat the subject while checking a situation of the inside of the body of the subject from the ultrasonic image. In this case, since the probe can be stopped at a desired position among multiple points, the probe can be stopped at an essential place, and the convenience of a probe diagnosis using the robot device can be improved.

An arm operating method of the present disclosure is an arm operating method of a robot device including an arm holding a probe of an ultrasonic diagnostic device and a force sensor provided on the arm, the robot device being for supporting a manual operation of the probe by an operator based on a signal from the force sensor and operating the arm to automatically operate the probe based on an instruction of the operator, the arm operating method including interpolating a trajectory between multiple random points registered in advance by the manual operation, and operating the arm such that the probe moves along the trajectory in the automatic operation and can stop at a desired position between the multiple points based on the instruction of the operator.

In the arm operating method of the present disclosure, the trajectory between multiple random points registered in advance by the manual operation is interpolated, and the arm is operated such that the probe moves along the trajectory in the automatic operation and can stop at a desired position between the multiple points based on an instruction of an operator.

### Brief Description of Drawings

Fig. 1 is an external perspective view of an ultrasonic diagnostic system including a robot device of the present embodiment.
Fig. 2 is a side view of the robot device.
Fig. 3 is a block diagram illustrating an electrical connection relationship between a robot device and an ultrasonic diagnostic device.
Fig. 4 is a flowchart illustrating one example of automatic operation mode processing.
Fig. 5 is a flowchart illustrating one example of setting processing.
Fig. 6 is an explanatory diagram illustrating one example of a setting screen.
Fig. 7 is a flowchart illustrating one example of interpolation processing.
Fig. 8 is an explanatory diagram illustrating one example of an interpolation method selection screen.
Fig. 9 is an explanatory diagram illustrating one example of an interpolation amount selection screen.
Fig. 10 is a flowchart illustrating one example of point addition processing.
Fig. 11 is a flowchart illustrating one example of sort processing.
Fig. 12 is an explanatory diagram illustrating one example of a sort condition selection screen.
Fig. 13 is a flowchart illustrating one example of fine-adjustment processing.
Fig. 14 is an explanatory diagram illustrating one example of an operation screen.

### Description of Embodiments

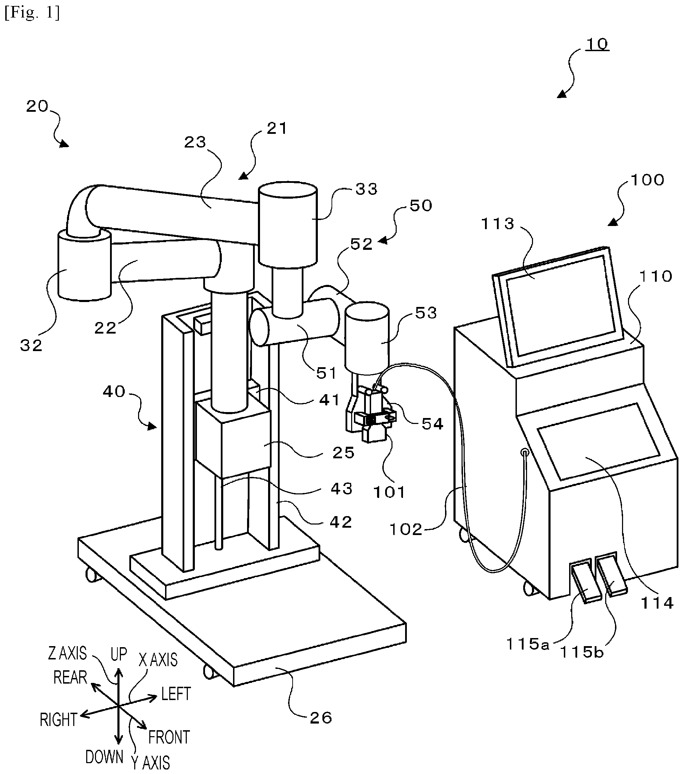

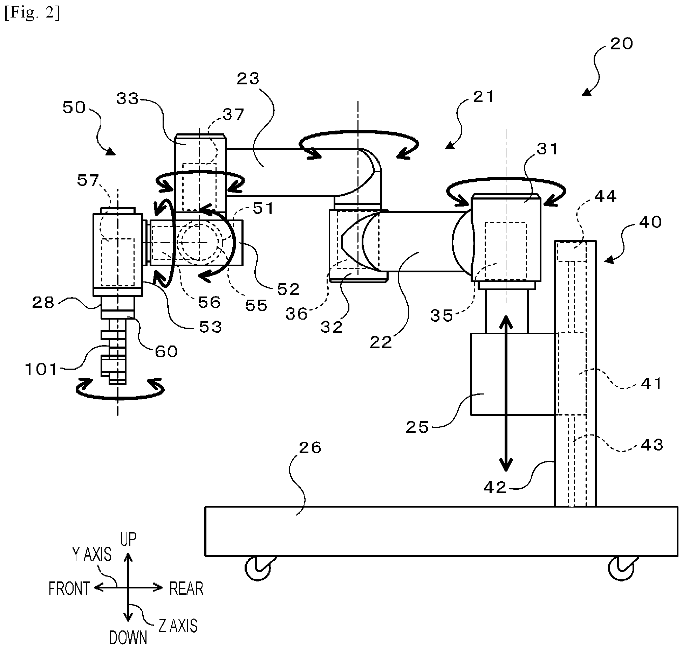

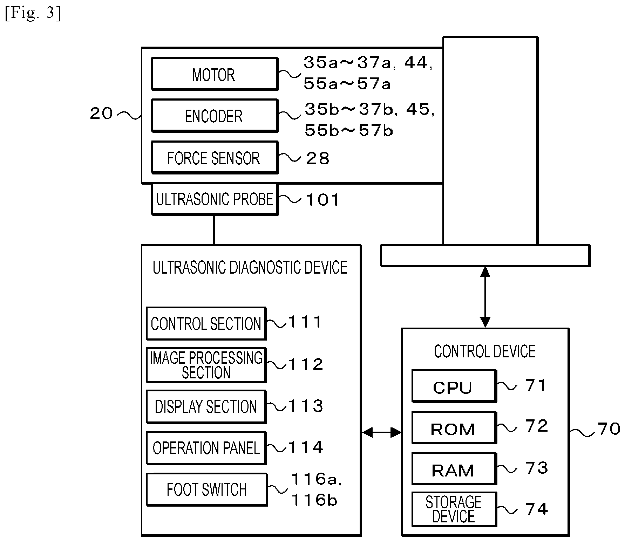

Hereinafter, embodiments of the present disclosure will be described with reference to the drawings. Fig. 1 is an external perspective view of ultrasonic diagnostic system 10 including robot device 20 of the present embodiment. Fig. 2 is a side view of robot device 20. Fig. 3 is a block diagram illustrating an electrical connection relationship between robot device 20 and ultrasonic diagnostic device 100. In Fig. 1, a left-right direction is an X axis, a front-rear direction is a Y-axis direction, and an up-down direction is a Z-axis direction.

Ultrasonic diagnostic system 10 of the present embodiment holds ultrasonic probe 101 on a hand of an arm of robot device 20, and operates robot device 20 such that ultrasonic probe 101 is pressed against a body surface of a patient, thereby acquiring an ultrasonic echo image. Ultrasonic diagnostic system 10 is used in, for example, catheter treatment. An operator who operates a guide wire of a catheter can press ultrasonic probe 101 against a body surface of a patient (subject) and operates the guide wire while recognizing a positional relationship between a front end of the guide wire and a blood vessel from the obtained ultrasonic echo image, thereby causing the guide wire to accurately pass through the center of an occluded portion or a stenosed portion of the blood vessel.

As illustrated in Figs. 1 to 3, ultrasonic diagnostic system 10 includes robot device 20 and ultrasonic diagnostic device 100.

Ultrasonic diagnostic device 100 includes ultrasonic probe 101 and ultrasonic diagnostic device body 110 coupled to ultrasonic probe 101 through cable 102. As illustrated in Fig. 3, ultrasonic diagnostic device body 110 includes control section 111 that controls the entire device, image processing section 112 that processes a reception signal from ultrasonic probe 101 to generate an ultrasonic echo image, display section 113 that displays the ultrasonic echo image, operation panel 114 that displays various types of information and can be operated by an operator, two foot pedals 115a and 115b, and foot switches 116a and 116b that detect stepping operations of foot pedals 115a and 115b by the operator.

As illustrated in Fig. 2, robot device 20 includes arm (robot arm) 21, force sensor 28 provided at a front end portion of arm 21, and control device 70 (see Fig. 3) that controls arm 21. Arm 21 is installed on base plate 26 and includes first arm 22, second arm 23, base 25, first arm drive device 35, second arm drive device 36, posture holding device 37, lifting and lowering device 40, rotation three-axis mechanism 50, and holder 60.

A base end portion of first arm 22 is coupled to base 25 through first joint shaft 31 extending in an up-down direction (Z-axis direction). First arm drive device 35 includes motor 35a and encoder 35b. A rotation shaft of motor 35a is coupled to first joint shaft 31 through a decelerator (not illustrated). First arm drive device 35 rotates (revolves) first arm 22 along a horizontal plane (XY plane) around first joint shaft 31 as motor 35a rotationally drives first joint shaft 31. Encoder 35b is attached to a rotation shaft of motor 35a and is configured as a rotary encoder that detects a rotation displacement amount of motor 35a.

A base end portion of second arm 23 is coupled to a front end portion of first arm 22 through second joint shaft 32 extending in the up-down direction. Second arm drive device 36 includes motor 36a and encoder 36b. A rotation shaft of motor 36a is coupled to second joint shaft 32 through a decelerator (not illustrated). Second arm drive device 36 rotates (revolves) second arm 23 along a horizontal plane around second joint shaft 32 as motor 36a rotationally drives second joint shaft 32. Encoder 36b is attached to a rotation shaft of motor 36a, and is configured as a rotary encoder that detects a rotation displacement amount of motor 36a.

Base 25 is provided so as to be lifted and lowered with respect to base plate 26 by lifting and lowering device 40 installed on base plate 26. As illustrated in Figs. 1 and 2, lifting and lowering device 40 includes slider 41 fixed to base 25, guide member 42 fixed to base plate 26 and extending in the up-down direction to guide movement of slider 41, ball screw shaft 43 (lifting and lowering shaft) extending in the up-down direction and screwed into a ball screw nut (not illustrated) fixed to slider 41, motor 44 for rotationally driving ball screw shaft 43, and encoder 45 (see Fig. 3). Lifting and lowering device 40 moves base 25 fixed to slider 41 in the up-down direction along guide member 42 by rotationally driving ball screw shaft 43 by using motor 44. Encoder 45 is configured as a linear encoder that detects a position (a lifting and lowering position) of slider 41 (base 25) in the up-down direction.

Rotation three-axis mechanism 50 is coupled to a front end portion of second arm 23 through posture holding shaft 33 extending in the up-down direction. Rotation three-axis mechanism 50 includes first rotation shaft 51, second rotation shaft 52, and third rotation shaft 53 that are orthogonal to each other, first rotation device 55 that rotates first rotation shaft 51, second rotation device 56 that rotates second rotation shaft 52, and third rotation device 57 that rotates third rotation shaft 53. First rotation shaft 51 is supported in a posture orthogonal to posture holding shaft 33. Second rotation shaft 52 is supported in a posture orthogonal to first rotation shaft 51. Third rotation shaft 53 is supported in a posture orthogonal to second rotation shaft 52. First rotation device 55 includes motor 55a for rotationally driving first rotation shaft 51, and encoder 55b that is attached to a rotation shaft of motor 55a and detects a rotation displacement amount of motor 55a. Second rotation device 56 includes motor 56a for rotationally driving second rotation shaft 52, and encoder 56b that is attached to a rotation shaft of motor 56a and detects a rotation displacement amount of motor 56a. Third rotation device 57 includes motor 57a for rotationally driving third rotation shaft 53, and encoder 57b that is attached to a rotation shaft of motor 57a and detects a rotation displacement amount of motor 57a. In addition, holder 60 for holding ultrasonic probe 101 is attached to third rotation shaft 53. In the present embodiment, ultrasonic probe 101 is held by holder 60 so as to be located coaxially with third rotation shaft 53.

Robot device 20 of the present embodiment can move ultrasonic probe 101 to a random position in any posture by combining a translational motion in three directions of the X-axis direction, the Y-axis direction, and the Z-axis direction by first arm drive device 35, second arm drive device 36, and lifting and lowering device 40 with a rotational motion in three directions of an X-axis spin (pitching), a Y-axis spin (rolling), and a Z-axis spin (yawing) by rotation three-axis mechanism 50.

Posture holding device 37 holds a posture (an orientation of first rotation shaft 51) of rotation three-axis mechanism 50 in a constant orientation regardless of the postures of first arm 22 and second arm 23. Posture holding device 37 includes motor 37a and encoder 37b. A rotation shaft of motor 37a is coupled to posture holding shaft 33 through a decelerator (not illustrated). Posture holding device 37 sets a target rotation angle of posture holding shaft 33 based on a rotation angle of first joint shaft 31 and a rotation angle of second joint shaft 32 such that an axial direction of first rotation shaft 51 is constantly in a left-right direction (the X-axis direction), and drives and controls motor 37a such that posture holding shaft 33 is at the target rotation angle. Thereby, the translational motion in the three directions and the rotational motion in the three directions can be independently controlled, and thus, control is easily made.

Force sensor 28 is attached to a front end of arm 21, and detects a force component acting in each axial direction of the X axis, the Y axis, and the Z axis as an external force acting on arm 21, and detects a torque component acting around each axis.

As illustrated in Fig. 3, control device 70 is configured with a microprocessor mainly including CPU 71, and includes ROM 72, RAM 73, nonvolatile storage device 74, input and output ports, and communication ports (not illustrated) in addition to CPU 71. A detection signal from force sensor 28, detection signals (position signals) from respective encoders 35b, 36b, 37b, 45, 55b, 56b, and 57b, and the like are input to control device 70 through input ports. Further, drive signals are output from control device 70 to respective motors 35a, 36a, 37a, 44, 55a, 56a, and 57a through output ports. Control device 70 communicates with control section 111 of ultrasonic diagnostic device 100 through communication ports, and exchanges various control signals and data with each other.

Next, an operation of robot device 20 of the present embodiment provided in ultrasonic diagnostic system 10 configured as described above will be described. Robot device 20 of the present embodiment has a manual operation mode in which an operator can manually operate ultrasonic probe 101 in a state where ultrasonic probe 101 is held by arm 21. In the manual operation mode, control device 70 of robot device 20 acquires the magnitude and direction of an external force detected by force sensor 28 when an operator manually operates ultrasonic probe 101, and performs an assist control for driving and controlling respective motors 35a, 36a, 37a, 44, 55a, 56a, and 57a such that an assist force corresponding to a magnitude of the acquired external force acts in a direction of the acquired external force. That is, robot device 20 of the present embodiment has a function of supporting an operator to manually operate ultrasonic probe 101.

Further, robot device 20 of the present embodiment also has a teaching mode in which a position and posture of ultrasonic probe 101 manually operated by an operator can be registered at multiple random points, and an automatic operation mode in which ultrasonic probe 101 is automatically moved so as to pass through multiple registered points in a predetermined order. In the teaching mode, and in the manual operation mode, control device 70 acquires position signals from respective encoders 35b, 36b, 37b, 45, 55b, 56b, and 57b based on an instruction of an operator, calculates a position and posture of ultrasonic probe 101 by forward kinematics based on the acquired position signals, and stores the calculated position and posture as registration points in storage device 74. The instruction of an operator can be executed by, for example, a stepping operation of foot pedal 115a, an operation of operation panel 114, an operation by voice recognition, and the like.

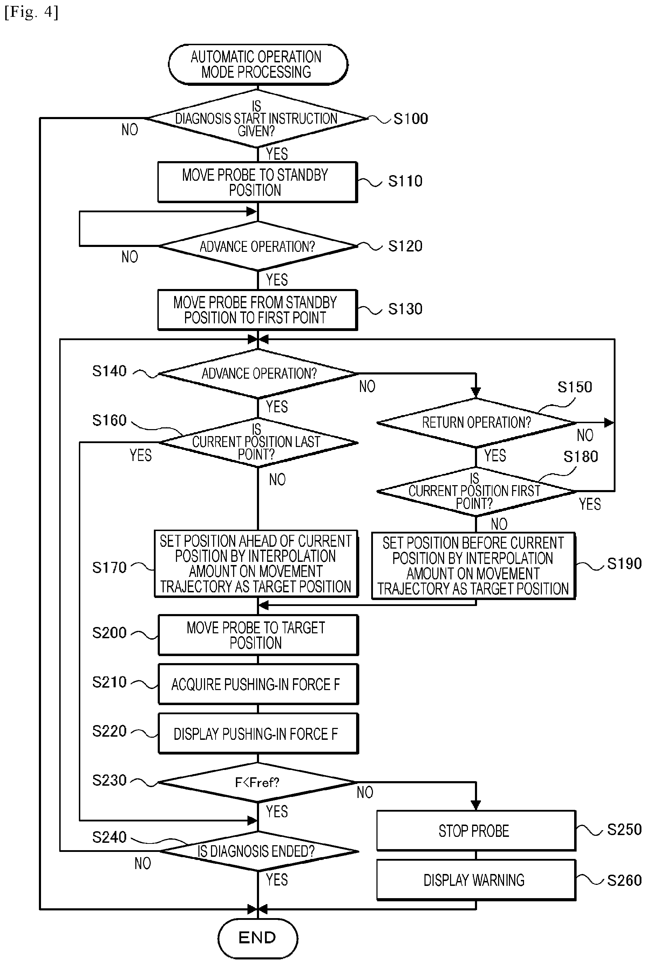

The automatic operation mode is performed by performing automatic operation mode processing. Fig. 4 is a flowchart illustrating one example of automatic operation mode processing performed by CPU 71 of control device 70.

When the automatic operation mode processing is performed, CPU 71 of control device 70 first determines whether a diagnosis start instruction is given by an operation of operation panel 114 (step S100). When it is determined that the diagnosis start instruction is not given, CPU 71 ends the automatic operation mode processing. Meanwhile, when it is determined that the diagnosis start instruction is given, CPU 71 controls respective motors such that ultrasonic probe 101 moves to a standby position (step S110). The standby position is set to a position separated upward by a predetermined distance from a first point among multiple points registered in advance. This is because, the body of a person has unevenness, and accordingly, when ultrasonic probe 101 is directly moved to the first point, ultrasonic probe 101 may come into contact (collide) with the body of the person at a point other than the first point.

Here, the movement of ultrasonic probe 101 is performed as follows. That is, CPU 71 first determines a target position and a target posture of arm 21 holding ultrasonic probe 101 at the hand. Subsequently, CPU 71 sets respective target rotation angles of first joint shaft 31, second joint shaft 32, posture holding shaft 33, first rotation shaft 51, second rotation shaft 52, and third rotation shaft 53 for moving arm 21 to a target position at a target posture and a target lifting and lowering position of base 25 by solving reverse kinetics based on the target position and the target posture. Then, CPU 71 controls a corresponding motor such that a rotation angle or a lifting and lowering position detected by each of encoders 35b, 36b, 37b, 45, 55b, 56b, and 57b coincides with a corresponding target rotation angle or a target lifting and lowering position.

When moving ultrasonic probe 101 to a standby position, then CPU 71 waits until an operator performs an advance operation (step S120). The advance operation can be performed by, for example, a stepping operation of foot pedal 115a, an operation of operation panel 114, an operation by voice recognition, or the like. In the present embodiment, CPU 71 determines that the advance operation is performed when foot switch 116a is turned on by the stepping operation of foot pedal 115a. When it is determined that the advance operation is performed, CPU 71 moves ultrasonic probe 101 from the standby position to the first point (step S130).

Next, CPU 71 determines whether the advance operation is performed (step S140) and whether a return operation is performed (step S150). The return operation can be performed by, for example, a stepping operation of foot pedal 115b, an operation of operation panel 114, an operation by voice recognition, or the like, by a different operation from the advance operation described above. In the present embodiment, ultrasonic diagnostic system 10 includes two foot pedals 115a and 115b, and the advance operation is performed by a stepping operation of foot pedal 115a, and the return operation is performed by a stepping operation of foot pedal 115b. When it is determined that neither the advance operation nor the return operation is performed, CPU 71 returns to step S140.

When it is determined in step S140 that the advance operation is performed, CPU 71 determines whether the current position of ultrasonic probe 101 is the last point among the multiple registered points (step S160). When it is determined that the current position is the last point, CPU 71 proceeds to step S240. Meanwhile, when it is determined that the current position is not the last point, CPU 71 sets a position and a posture ahead of the current position by an interpolation amount on a movement trajectory as a target position and a target posture (step S170). The movement trajectory is interpolated between multiple points by an interpolation method selected in advance. The interpolation amount is a unit movement amount of ultrasonic probe 101. Details of the movement trajectory and the interpolation amount will be described below. Then, CPU 71 sets a target rotation angle of each joint, such as first joint shaft 31, and a target lifting and lowering position of base 25 such that ultrasonic probe 101 moves to a target position at a target posture, and controls a corresponding motor (step S200).

When it is determined in step S150 that the return operation is performed, CPU 71 determines whether the current position of ultrasonic probe 101 is the first point (step S180). When it is determined that the current position is the first point, CPU 71 returns to step S140. Meanwhile, when it is determined that the current position is not the first point, CPU 71 sets a position and a posture before the current position by an interpolation amount on a movement trajectory as a target position and a target posture (step S180). Then, CPU 71 sets a target rotation angle of each joint, such as first joint shaft 31, and a target lifting and lowering position of base 25 such that ultrasonic probe 101 moves to a target position at a target posture, and controls a corresponding motor (step S200).

Next, CPU 71 acquires pushing-in force F of ultrasonic probe 101 against a body surface of a subject based on a detection signal from force sensor 28 (step S210). Subsequently, CPU 71 displays acquired pushing-in force F on display section 113 or operation panel 114 (step S220), and determines whether pushing-in force F is less than threshold Fref (step S230). Threshold Fref is an upper limit value of a pushing-in force that is allowed when ultrasonic probe 101 is pushed into a body surface of a subject, and is determined in advance by an experiment or the like, for example, as a force that does not cause the subject to feel pain or discomfort. When it is determined that pushing-in force F is less than threshold Fref, CPU 71 determines whether a diagnosis is ended (step S240). When it is determined that the diagnosis is not end, CPU 71 returns to step S140. Meanwhile, when it is determined that the diagnosis is ended, the automatic operation mode processing ends.

When an ultrasonic diagnosis is applied to catheter treatment, an operator performs teaching in advance such that a scan direction of ultrasonic probe 101 coincides with a central axis direction (longitudinal direction) of a blood vessel of a subject, and thereby, ultrasonic probe 101 can be automatically moved in the central axis direction of the blood vessel only by operating foot pedal 115a. Thereby, an operator can move a guide wire forward by him/herself bwhile checking a position of a blood vessel of a subject from an ultrasonic echo image by operating ultrasonic probe 101. At this time, since robot device 20 moves ultrasonic probe 101 by an interpolation amount (unit movement amount) each time foot pedal 115a is operated once, an operator can easily move ultrasonic probe 101 to a target position and concentrate on an operation of a guide wire.

When it is determined in step S170 that pushing-in force F is greater than or equal to threshold Fref, CPU 71 moves ultrasonic probe 101 upward by a predetermined distance and stops (emergently stops) (step S250). Then, CPU 71 displays a warning on display section 113 or operation panel 114 (step S260), and ends the automatic operation mode processing.

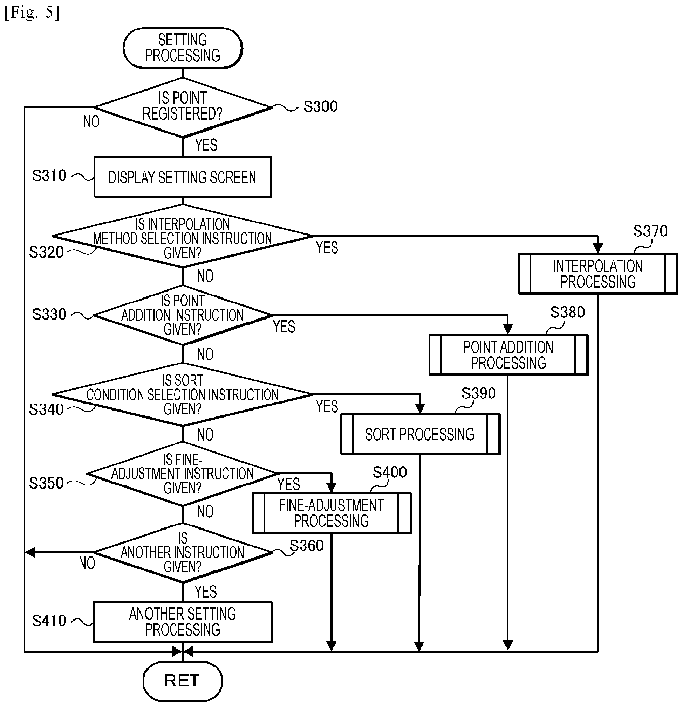

Next, setting processing for performing various settings in the automatic operation mode will be described. Fig. 5 is a flowchart illustrating one example of setting processing performed by CPU 71 of control device 70.

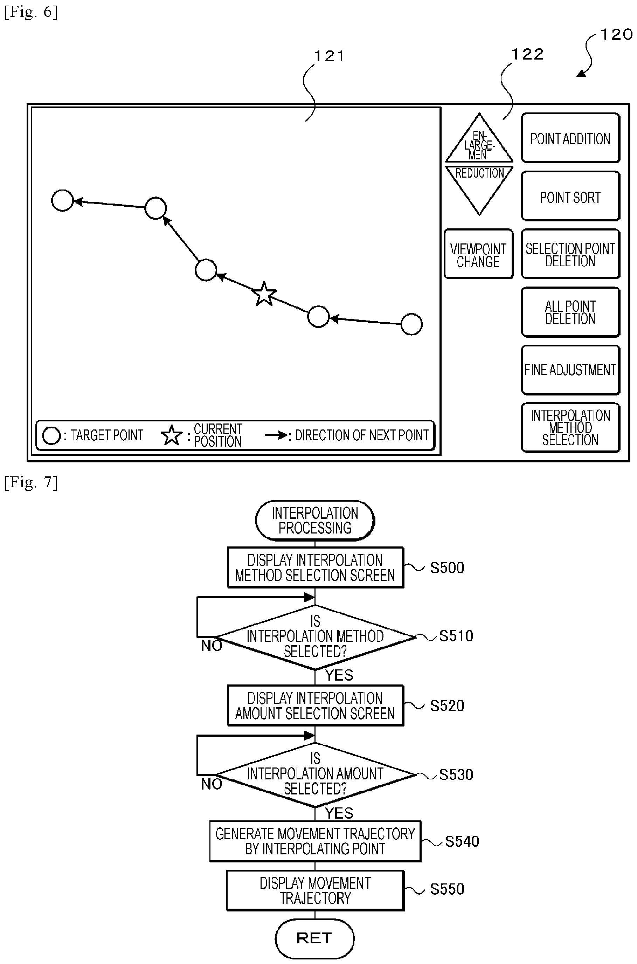

When the setting processing is performed, CPU 71 of control device 70 first determines whether multiple points are registered in storage device 74 (step S300). When it is determined that the registration is not completed, CPU 71 ends the setting processing. Meanwhile, when it is determined that the setting processing is registered, CPU 71 displays setting screen 120 on operation panel 114 (step S310). Fig. 6 is an explanatory diagram illustrating one example of setting screen 120. As illustrated in Fig. 6, setting screen 120 is provided with movement trajectory display section 121 that displays a movement trajectory of ultrasonic probe 101 passing through multiple registered points, and setting button display section 122 that displays various setting buttons. Movement trajectory display section 121 three-dimensionally displays a movement path of ultrasonic probe 101 so as to pass through multiple points (target points) that are registered in advance. Movement trajectory display section 121 includes multiple registered target points, a current position of ultrasonic probe 101, and a movement trajectory and a movement direction (arrow) from each target point to a next target point except for an end point. Setting button display section 122 displays buttons for selecting various settings when automatically moving ultrasonic probe 101 in the automatic operation mode described above. In the present embodiment, setting button display section 122 includes an enlargement/reduction button, a display viewpoint change button, a point addition button, a point sort button, a selection point deletion button, an all-point deletion button, a point fine-adjustment button, and an interpolation method selection button. The enlargement/reduction button is for instructing enlargement or reduction of display in movement trajectory display section 121. The display viewpoint change button is for instructing a change of a display viewpoint (a console viewpoint, a right-overhead viewpoint, or the like) in movement trajectory display section 121. The point addition button is for instructing addition of a new point. The point sort button is for instructing a change of a movement order of ultrasonic probe 101 for multiple registered points. The selection point deletion button is for instructing deletion of some of the multiple registered points. The all-point deletion button is for instructing deletion of all registered points. The point fine-adjustment button is for instructing fine adjustment of a position of the registered point. The interpolation method change button is for instructing a change of an interpolation method for interpolating a trajectory between registered points.

When setting screen 120 is displayed, CPU 71 determines whether a selection instruction of an interpolation method is given by an operation of the interpolation method selection button (step S320), whether an addition instruction of a point is given by an operation of the point addition button (step S330), whether a selection instruction of a sort condition is given by an operation of the point sort button (step S340), whether a fine-adjustment instruction of a point is given by an operation of the fine-adjustment button (step S350), and whether another instruction is given (step S360).

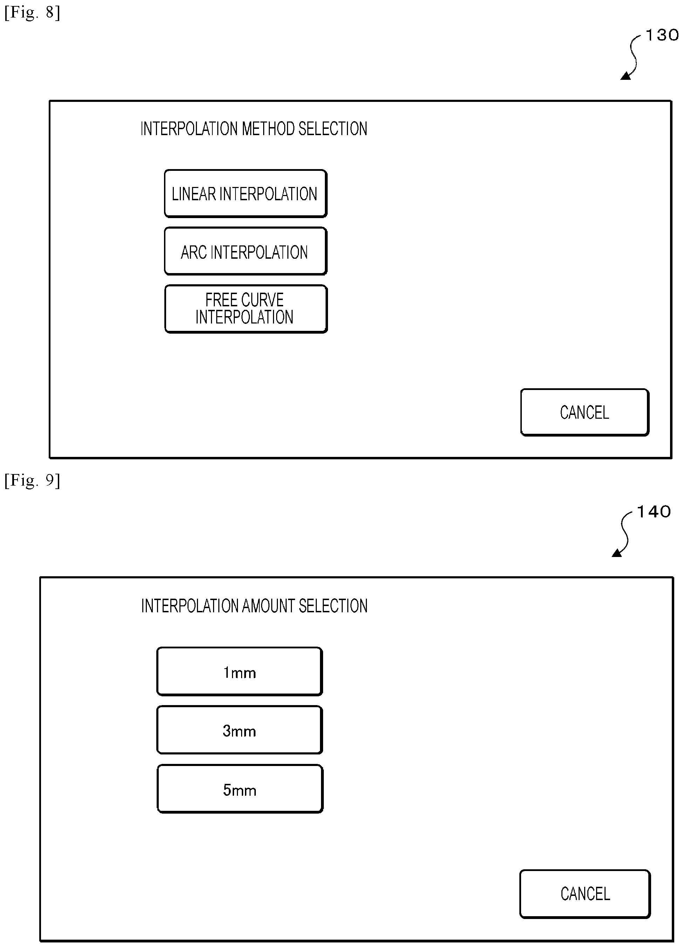

When it is determined in step S320 that an interpolation method selection instruction is given, CPU 71 performs interpolation processing (step S370), and ends the setting processing. Fig. 7 is a flowchart illustrating one example of interpolation processing. In the interpolation processing, CPU 71 first displays interpolation method selection screen 130 (step S500), and determines whether an interpolation method is selected (step S510). Fig. 8 is an explanatory diagram illustrating one example of interpolation method selection screen 130. As illustrated, interpolation method selection screen 130 includes, for example, linear interpolation, arc interpolation, and free curve interpolation as selectable interpolation methods. When it is determined that the interpolation method is selected, CPU 71 displays interpolation amount selection screen 140 (step S520), and determines whether an interpolation amount is selected (step S530). Fig. 9 is an explanatory diagram illustrating one example of interpolation amount selection screen 140. The interpolation amount is a unit movement amount by which ultrasonic probe 101 moves in response to one instruction (one stepping operation of foot pedals 115a and 115b) of an operator. When it is determined that the interpolation method and the interpolation amount are selected, CPU 71 generates a movement trajectory by interpolating between points by the selected interpolation method (step S540), displays the generated movement trajectory on movement trajectory display section 121 of setting screen 120 (step S550), and ends the interpolation processing.

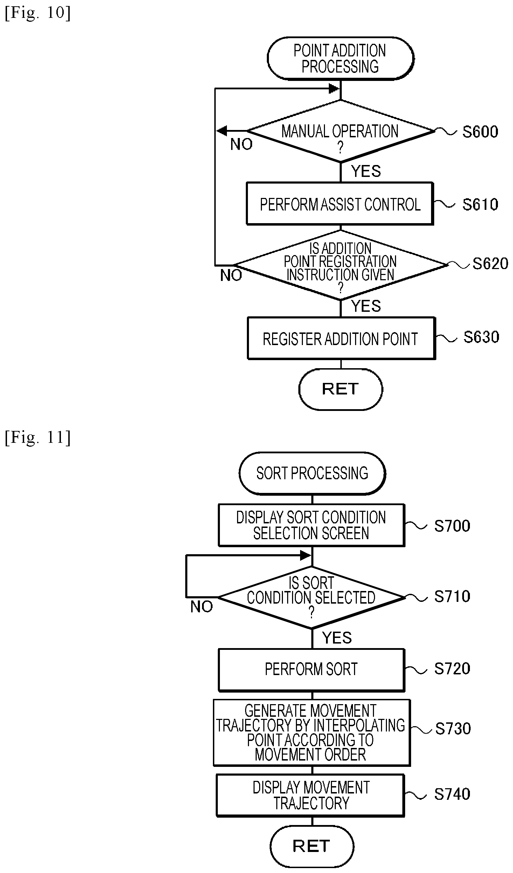

When it is determined in step S330 that there is an instruction to add a point, CPU 71 performs point addition processing (step 380), and ends the setting processing. Fig. 10 is a flowchart illustrating one example of point addition processing. In the point addition processing, CPU 71 waits for an operator to manually operate arm 21 based on a detection signal from force sensor 28 (step S600). When it is determined that arm 21 is manually operated, CPU 71 performs the above-described assist control (step S610), and determines whether an addition point registration instruction is given (step S620). When it is determined that the addition point registration instruction is not given, CPU 71 returns to step S600, and when it is determined that the addition point registration instruction is given, CPU 71 registers the addition point (step S630), and ends the point addition processing. The registration of addition point is performed by acquiring position signals from respective encoders 35b, 36b, 37b, 45, 55b, 56b, and 57b, calculating a current position and posture of ultrasonic probe 101 by forward kinematics based on the acquired position signals, and storing the calculated position and posture as the addition point in storage device 74.

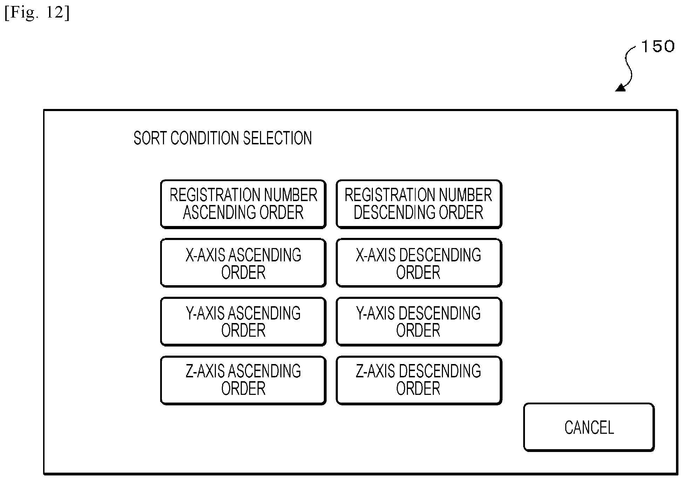

When it is determined in step S340 that a sort condition selection instruction is given, CPU 71 performs sort processing (step S390) and ends the setting processing. Fig. 11 is a flowchart illustrating one example of sort processing. In the sort processing, CPU 71 first displays sort condition selection screen 150 (step S700) and determines whether the sort condition is selected (step S710). Fig. 12 is an explanatory diagram illustrating one example of sort condition selection screen 150. As illustrated, sort condition selection screen 150 includes a registration number ascending order, a registration number descending order, an X-axis ascending order, an X-axis descending order, a Y-axis ascending order, a Y-axis descending order, a Z-axis ascending order, and a Z-axis descending order. In the registration number ascending order, movement orders are rearranged from the oldest to the latest based on registration timing. In the registration number descending order, movement orders are rearranged from the latest to the oldest based on registration timing. In the X-axis ascending order, movement orders are rearranged from the largest to the smallest in the X-axis direction. In the X-axis descending order, movement orders are rearranged from the smallest to the largest in the X-axis direction. In the Y-axis ascending order, movement orders are rearranged from the largest to the smallest in the Y-axis direction. In the Y-axis descending order, movement orders are rearranged from the smallest to the largest in the Y-axis direction. In the Z-axis ascending order, movement orders are rearranged from the largest to the smallest in the Z-axis direction. In the Z-axis descending order, movement orders are rearranged from the smallest to the largest in the Z-axis direction. When the sort condition is selected, CPU 71 sorts multiple registered points by the selected sort condition (step S720). Then, CPU 71 generates a movement trajectory by interpolating the multiple sorted points by the currently selected interpolation method (step S730), updates the display of movement trajectory display section 121 by using the generated movement trajectory (step S740), and ends the sort processing.

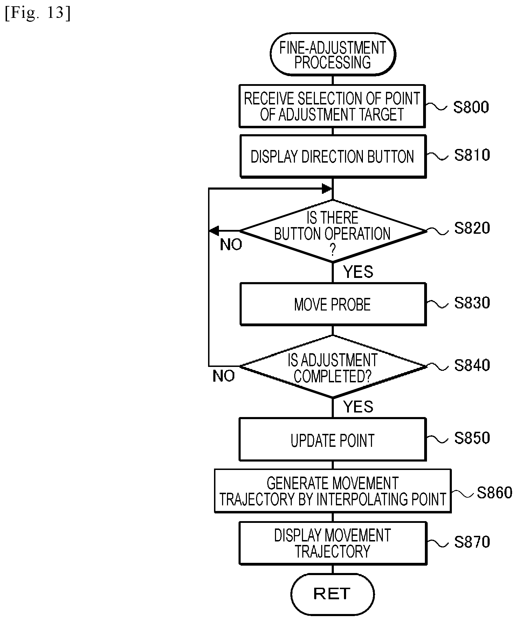

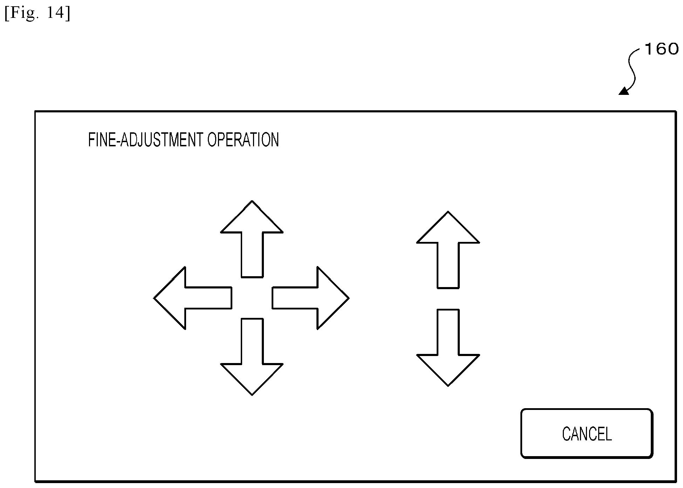

When it is determined in step S350 that a fine-adjustment instruction is given, CPU 71 performs fine-adjustment processing (step S400) and ends the setting processing. Fig. 13 is a flowchart illustrating one example of fine-adjustment processing. In the fine-adjustment processing, CPU 71 first receives selection of a point of an adjustment target (step S800). Next, CPU 71 displays operation screen 160 including multiple direction buttons on operation panel 114 (step S810) and determines whether the direction button is operated (step S820). Fig. 14 is an explanatory diagram illustrating one example of operation screen 160. As illustrated, operation screen 160 includes front, rear, left, and right direction buttons and up and down direction buttons. A fine-adjustment instruction can be performed by an operation of operation panel 114, an operation by using a joystick (not illustrated), and the like. When it is determined that any of the direction buttons is operated, CPU 71 controls arm 21 such that ultrasonic probe 101 moves by a predetermined amount in a direction corresponding to the operated direction button (step S830). The predetermined amount may be set to a predetermined amount or may be set to be longer or shorter according to an operation time of the direction button. Then, CPU 71 determines whether adjustment completion instruction is given (step S840). When it is determined that the adjustment completion instruction is not given, CPU 71 returns to step S820. Meanwhile, when it is determined that the adjustment completion instruction is given, CPU 71 updates the point (step S850). The point is updated by acquiring position signals from respective encoders 35b, 36b, 37b, 45, 55b, 56b, and 57b, calculating a current position and posture of ultrasonic probe 101 by using forward kinematics based on the acquired position signals, and replacing the calculated position and posture with the points before adjustment as the points after adjustment. Then, CPU 71 generates a movement trajectory by interpolating multiple registered points including the finely adjusted points by using the currently set interpolation method (step S860), updates the display of movement trajectory display section 121 by using the generated movement trajectory (step S870), and ends the setting processing.

When it is determined in step S360 that another setting instruction is given, CPU 71 performs another setting processing (step S410), and ends the setting processing. Another setting processing can include, for example, processing of displaying a movement trajectory on movement trajectory display section 121 according to the instructed viewpoint when a viewpoint change instruction is given, processing of deleting points thereof when a point deletion instruction is given, and the like.

Here, a correspondence relationship between main elements of the embodiment and main elements of the present disclosure described in the scope of claims will be described. That is, ultrasonic diagnostic device 100 of the present embodiment corresponds to an ultrasonic diagnostic device, ultrasonic probe 101 corresponds to a probe, arm 21 corresponds to an arm, force sensor 28 corresponds to a force sensor, robot device 20 corresponds to a robot device, storage device 74 corresponds to a storage device, and control device 70 corresponds to a control device.

The present disclosure is not limited in any way to the embodiments described above, and it is needless to say that the present disclosure can be embodied in various aspects as long as the various aspects fall within the technical scope of the present disclosure.

For example, in the embodiments described above, robot device 20 is configured as a seven-axis articulated robot capable of performing a translational motion in three directions and a rotational motion in three directions. However, the number of axes may be any number. Further, robot device 20 may be implemented by a so-called vertical articulated robot, a horizontal articulated robot, or the like.

As described above, the robot device of the present disclosure stores a position and posture of a probe for each point at multiple random points by a manual operation. Then, the robot device interpolates a trajectory between the multiple points, and the probe moves along the trajectory in an automatic operation. Accordingly, an operator can deal with the treatment of a subject by him/herself while checking the situation in the body of the subject from an ultrasonic image by operating the probe. In this case, since the probe can be stopped at a desired position among multiple points, the probe can be stopped at an essential place, and the convenience of a probe diagnosis using the robot device can be improved.

In the robot device according to the present disclosure, the control device may receive selection of the operator from among multiple predetermined interpolation methods and interpolate the trajectory between the multiple points by using the selected interpolation method. Accordingly, it is possible to move the probe along a desired movement trajectory between multiple points.

In the robot device of the present disclosure, the control device may set a unit movement amount of the probe based on the instruction of the operator and operate the arm such that the probe moves between the multiple points by the unit movement amount based on the instruction of the operator. Accordingly, it is possible to move the probe at a desired pace.

Furthermore, in the robot device of the present disclosure, the control device may perform position adjustment to operate the arm such that the probe moves from a position on the trajectory to a random position based on the instruction of the operator. Accordingly, it is possible to correct the movement trajectory of the probe later.

The present disclosure is not limited to the embodiment of a robot device and can be implemented by the embodiment of an arm operating method.

### Industrial Applicability

The present disclosure can be applied to a robot device manufacturing industry and the like.

### Reference Signs List

10 ultrasonic diagnostic system, 20 robot device, 21 arm, 22 first arm, 23 second arm, 25 base, 26 base plate, 28 force sensor, 31 first joint shaft, 32 second joint shaft, 33 posture holding shaft, 35 first arm drive device, 35a motor, 35b encoder, 36 second arm drive device, 36a motor, 36b encoder, 37 posture holding device, 37a motor, 37b encoder, 40 lifting and lowering device, 41 slider, 42 guide member, 43 ball screw shaft, 44 motor, 45 encoder, 50 rotation three-axis mechanism, 51 first rotation shaft, 52 second rotation shaft, 53 third rotation shaft, 55 first rotation device, 55a motor, 55b encoder, 56 second rotation device, 56a motor, 56b encoder, 57 third rotation device, 57a motor, 57b encoder, 60 holder, 70 control device, 71 CPU, 72 ROM, 73 RAM, 74 storage device, 100 ultrasonic diagnostic device, 101 ultrasonic probe, 102 cable, 110 ultrasonic diagnostic device body, 111 control section, 112 image processing section, 113 display section, 114 operation panel, 115a,115b foot pedal, 116a,116b foot switch, 120 setting screen, 121 movement trajectory display section, 122 setting button display section, 130 interpolation method selection screen, 140 interpolation amount selection screen, 150 sort condition selection screen, 160 operation screen

## Claims

1. A robot device comprising:
an arm holding a probe of an ultrasonic diagnostic device;
a force sensor provided on the arm, the robot device being for supporting a manual operation of the probe by an operator based on a signal from the force sensor and operating the arm to automatically operate the probe based on an instruction of the operator;
a storage device configured to store a position and a posture of the probe for each point among multiple random points by the manual operation; and
a control device configured to interpolate a trajectory between the multiple points stored in the storage device, and operate the arm such that the probe moves along the trajectory in the automatic operation and can stop at a desired position between the multiple points based on the instruction of the operator.

2. The robot device according to Claim 1, wherein
the control device receives selection of the operator from among multiple predetermined interpolation methods and interpolates the trajectory between the multiple points by using the selected interpolation method.

3. The robot device according to Claim 1 or 2, wherein
the control device sets a unit movement amount of the probe based on the instruction of the operator and operates the arm such that the probe moves between the multiple points by the unit movement amount based on the instruction of the operator.

4. The robot device according to any one of Claims 1 to 3, wherein
the control device performs position adjustment to operate the arm such that the probe moves from a position on the trajectory to a random position based on the instruction of the operator.

5. An arm operating method of a robot device comprising an arm holding a probe of an ultrasonic diagnostic device and a force sensor provided on the arm, the robot device being for supporting a manual operation of the probe by an operator based on a signal from the force sensor and operating the arm to automatically operate the probe based on an instruction of the operator, the arm operating method comprising:
interpolating a trajectory between multiple random points registered in advance by the manual operation, and operating the arm such that the probe moves along the trajectory in the automatic operation and can stop at a desired position between the multiple points based on the instruction of the operator.
